(19) Europäisches Patentamt
European Patent Office
Office européen des brevets

(11) EP 4 803 116 A1

(12) **EUROPEAN PATENT APPLICATION**
published in accordance with Art. 153(4) EPC

(43) Date of publication:
**09.09.2026 Bulletin 2026/37**

(21) Application number: **24885565.2**

(22) Date of filing: **23.10.2024**

(51) International Patent Classification (IPC):
***A61M 1/36** (2006.01)* ***B01J 20/26** (2006.01)*

(52) Cooperative Patent Classification (CPC):
**A61M 1/36; B01J 20/26**

(86) International application number:
**PCT/JP2024/037771**

(87) International publication number:
**WO 2025/094792 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 JP 2023186058**

(71) Applicant: **Toray Industries, Inc.**
**Tokyo 103-8666 (JP)**

(72) Inventors:
- **YAMASHITA, Kyohei**
**Otsu-shi, Shiga 520-8558 (JP)**
- **KANDA, Shungo**
**Otsu-shi, Shiga 520-8558 (JP)**
- **KOMACHI, Shunsuke**
**Otsu-shi, Shiga 520-8558 (JP)**
- **SEKIYA, Yumiko**
**Otsu-shi, Shiga 520-8558 (JP)**
- **TAKAHASHI, Hiroshi**
**Otsu-shi, Shiga 520-8558 (JP)**

(74) Representative: **Kador & Partner Part mbB**
**Corneliusstraße 15**
**80469 München (DE)**

(54) **BLOOD PURIFICATION MATERIAL**

(57) The purpose of the present invention is to provide a blood purification material that is capable of adsorbing and removing multiple inflammatory cytokines having different surface charges at high efficiency. The present invention provides a blood purification material having a water-insoluble base material, a ligand containing an acidic functional group, and a ligand containing a basic functional group. The ligand containing the acidic functional group and the ligand containing the basic functional group are bonded to the water-insoluble base material, and the value (MwA/MwB) obtained by dividing the molecular weight (MwA) of the ligand containing the acidic functional group by the molecular weight (MwB) of the ligand containing the basic functional group is less than 1.000.



Processed by Luminess, 75001 PARIS (FR)

## Description

### Technical Field

The present invention relates to a blood purification material.

### Background Art

In recent years, various blood purification materials and blood purification columns packed with the materials have been developed for the purpose of selectively separating blood components such as inflammatory cytokines from blood and adsorbing them.

Examples of commonly known methods to improve the adsorption performance of blood purification materials for blood components include to use, as a base material, a material that interacts strongly with the target substance (e.g., inflammatory cytokines), or to provide a ligand that interacts strongly with the target substance to the surface of the base material.

For example, Patent Literature 1 discloses that a material containing dextran sulfate and tryptophan on the surface of beads made of cellulose suppresses the complement activating activity of blood-contacting material and reduces the increase in plasma C3a concentration.

Patent Literature 2 discloses an adsorption material for blood components having a modified polymer in which a compound containing an acidic functional group is bonded via an amide bond to the surface of a water-insoluble a base material, and also discloses that cytokines can be adsorbed at high efficiency by specifying the water content of the modified polymer and the amount of acidic functional group introduced.

Patent Literature 3 discloses that a hollow fiber membrane, made of acrylonitrile and anionic monomer and surface-treated with cationic polymer, suppresses activation of the contact phase, which can cause anaphylactoid reactions.

For example, it is known that in patients with inflammatory diseases such as acute respiratory distress syndrome (ARDS) or sepsis, a phenomenon known as cytokine storm occurs, resulting in the excessive production of multiple inflammatory cytokines, represented by interleukin-6 (IL-6) and interleukin-8 (IL-8). These inflammatory cytokines vary in molecular weight from 5 to 20 kDa and have different surface charges, and it is desirable to eliminate such multiple cytokines in order to treat patients with inflammatory diseases.

### Citation List

#### Patent Literature

Patent Literature 1: JP 5374064
Patent Literature 2: JP 2023-121738 A
Patent Literature 3: JP 4579916 B

### Summary of Invention

#### Technical Problem

The purpose of the present invention is to provide a blood purification material that is capable of adsorbing and removing multiple inflammatory cytokines having different surface charges at high efficiency.

#### Solution to Problem

In order to solve the above problem, the present inventors have studied intensively and, as a result, found that a blood purification material in which the mass average molecular weight of ligands containing acidic functional groups is smaller than the mass average molecular weight of ligands containing basic functional groups can adsorb multiple inflammatory cytokines having different surface charges at high efficiency, and have completed the present invention.

That is, the present invention provides the following (1) to (7).

(1) A blood purification material having a water-insoluble base material, a ligand containing an acidic functional group, and a ligand containing a basic functional group, wherein the ligand containing the acidic functional group and the

ligand containing the basic functional group are bonded to the water-insoluble base material, and the value (MwA/MwB) obtained by dividing the mass average molecular weight (MwA) of the ligand containing the acidic functional group by the mass average molecular weight (MwB) of the ligand containing the basic functional group is less than 1.000.

(2) The blood purification material according to (1), wherein the MwA/MwB is 0.001 to 0.430.

(3) The blood purification material according to (1) or (2), wherein the MwA is $0.5 \times 10^2$ to $90.0 \times 10^3$, and the MwB is $1.0 \times 10^2$ to $66.6 \times 10^3$.

(4) The blood purification material according to any one of (1) to (3), wherein the content of the acidic functional group is 0.02 to 3.00 mmol per gram of dry weight, and the content of the basic functional group is 0.50 to 2.00 mmol per gram of dry weight.

(5) The blood purification material according to any one of (1) to (3), wherein the content of the acidic functional group is 0.02 to 1.50 mmol per gram of dry weight, and the content of the basic functional group is 0.70 to 1.50 mmol per gram of dry weight.

(6) The blood purification material according to any one of (1) to (5), for use in adsorbing inflammatory cytokines.

(7) A blood purification column comprising the blood purification material according to any one of (1) to (6).

Advantageous Effects of Invention

The blood purification material of the present invention is capable of adsorbing and removing multiple inflammatory cytokines having different surface charges at high efficiency.

Description of Embodiments

The blood purification material of the present invention is characterized in that it has a water-insoluble base material, a ligand containing an acidic functional group, and a ligand containing a basic functional group, the ligand containing the acidic functional group and the ligand containing the basic functional group are bonded to the surface of the water-insoluble base material, and the value (MwA/MwB) obtained by dividing the mass average molecular weight (MwA) of the ligand containing the acidic functional group by the mass average molecular weight (MwB) of the ligand containing the basic functional group is less than 1.000.

The term "blood purification material" refers to a material that adsorbs organic substances present in blood components, and includes a water-insoluble base material in at least a part of the material. Examples of the material include those including a water-insoluble base material alone and those including a water-insoluble base material with a suitable reinforcing material immobilized thereto or mixed therein. The immobilization or mixing operation may be performed before or after processing into a desired shape.

The term "blood components" refers to components that constitute blood, and example thereof include humoral factors in blood and cells in blood. There is no particular restriction on the blood components that are substances to be adsorbed by the blood purification material of the present invention, but among the blood components, liquid factors in blood are preferred as the substances to be adsorbed.

The term "humoral factor in blood" means an organic substance dissolved in blood. Specific examples include urea, β2-microglobulin, inflammatory cytokines, proteins such as IgE or IgG, and polysaccharides such as lipopolysaccharide (hereinafter abbreviated as LPS). Among them, urea, proteins such as inflammatory cytokines, or polysaccharides such as LPS are preferred as substances to be adsorbed, and when the blood purification material of the present invention is used for the purpose of treating inflammatory diseases, inflammatory cytokines are more preferred as the substances to be adsorbed.

The term "inflammatory cytokine" refers to a group of proteins that are produced from various cells, including immunocompetent cells, in response to stimuli such as infection or trauma, and are released extracellularly to act, and examples thereof include interferon α, interferon β, interferon γ, interleukin 1 to interleukin 15, tumor necrosis factor α, tumor necrosis factor β, high mobility group box-1, erythropoietin, and monocyte chemotactic factor. Among them, inflammatory cytokines are classified into positively charged cytokines and negatively charged cytokines based on the difference in their surface charges.

The "positively charged cytokines" are cytokines with an isoelectric point of 7.5 or more, and examples thereof include interleukin 8 (hereinafter referred to as IL-8) and interferon β.

The "negatively charged cytokines" are cytokines with an isoelectric point of 6.5 or less, and examples thereof include interleukin 6 (hereinafter referred to as IL-6) and interleukin 12 (IL-12).

The blood purification material according to this embodiment is preferably for use in adsorbing proteins, and more preferably for use in adsorbing cytokines. Among cytokines, IL-8 and IL-6 are major causative substances of inflammatory diseases, and therefore, the blood purification material is more preferably for use in adsorbing IL-8 and/or IL-6, and the most preferably for use in adsorbing IL-8 and IL-6.

The term "adsorption" means a state in which a substance adheres to a material and is not easily detached, or an adsorption equilibrium state. The principle of adsorption is not particularly limited, but examples thereof include intermolecular forces such as electrostatic interactions, hydrophobic interactions, hydrogen bonds, and van der Waals forces.

The term "water-insoluble base material" means a base material that is insoluble in water. Insoluble in water means that the change in dry weight before and after the water-insoluble base material is placed in water is 1% or less. The change in dry weight is a ratio of the dry weight of the solid content remaining after immersing the water-insoluble base material in water at 37°C in an amount 9 times the dry weight of the base material for 1 hour, then pulling up the base material with tweezers or the like, and vacuum-drying the remaining water at 50°C or less, to the dry weight of the water-insoluble base material before immersion. When the base material is not insoluble in water, there is a risk that the amount of eluted substances will increase during use, which is undesirable from a safety standpoint.

The term "dry weight" means the weight of the solid in its dry state. A solid in a dry state refers to a solid in which the amount of liquid component contained in the solid is 1% by weight or less. After the weight of the solid is measured, the solid is dried by heating at 80°C and atmospheric pressure for 24 hours. When the weight loss of the remaining solid is 1% by weight or less of its weight before drying, the solid is considered to be in a dry state.

Examples of the component constituting the water-insoluble base material include compounds selected from the group consisting of polyethylene terephthalate, polybutylene terephthalate, polyvinyl aromatic compound, polyester, polysulfone, polyethersulfone, polystyrene and derivatives thereof (e.g., polycarbonate, polyetherketone, polyetheretherketone, polyphenylene sulfide, polyphenol, polyphenylene ether, polyphenylene ethylene, polyamideimide, polystyrene sulfonate, poly(4-methylstyrene), poly(4-ethylstyrene), poly(4-isopropylstyrene), poly(2-chlorostyrene), poly(4-chlorostyrene), poly(3-hydroxystyrene), poly(4-methoxystyrene), poly(4-carboxystyrene), poly(4-nitrostyrene), poly(4-chloromethylstyrene), poly(2,4-dimethylstyrene), poly(2,5-dichlorostyrene), poly(2,4,5-tribromostyrene), poly(2,3,4,5,6-pentafluorostyrene), sulfonated polysulfone, sulfonated polyethersulfone), polyvinyl alcohol, cellulose acetate, polyacrylonitrile, as well as homopolymers, copolymers, and mixture thereof. When an acidic functional group or a basic functional group is to be immobilized on the surface of a water-insoluble base material, because of large number of aromatic rings per unit weight and ease of immobilization of sulfate, sulfonate, and amino groups, the component constituting the water-insoluble base material is preferably a compound selected from the group consisting of polystyrene, polystyrene derivatives, polysulfone, polysulfone derivatives, polyethersulfone, and polyethersulfone derivatives, and mixtures thereof, more preferably a compound selected from the group consisting of polystyrene, polystyrene derivatives, polysulfone, polysulfone derivatives, and mixtures thereof, and even more preferably polystyrene. Examples of the polystyrene derivative include polystyrene sulfonic acid, poly(4-methylstyrene), poly(4-ethylstyrene), poly(4-isopropylstyrene), poly(2-chlorostyrene), poly(4-chlorostyrene), poly(3-hydroxystyrene), poly(4-methoxystyrene), poly(4-carboxystyrene), poly(4-nitrostyrene), poly(4-chloromethylstyrene), poly(2,4-dimethylstyrene), and poly(2,5-dichlorostyrene). Examples of the polysulfone derivative include sulfonated polysulfone, and examples of the polyethersulfone derivative include sulfonated polyethersulfone.

The water-insoluble base material is preferably in the form of a fiber or particle, which has a large specific surface area and is easy to handle.

When the water-insoluble base material is in the form of a fiber, the form of the water-insoluble base material is preferably a yarn bundle, a yarn, a net, a knitted fabric, a woven fabric, a felt, a net, or the like, made from processed fibers, and more preferably a yarn bundle, a knitted fabric, a woven fabric, a felt, or a net, having a large specific surface area and a small flow path resistance. Among them, a knitted fabric, a felt, and a net can be produced by a known method using a fiber as a raw material. Examples of the method of producing knitted fabrics and nets include a plain weaving method and a tubular knitting method. In particular, from the viewpoint of packing into a blood purifier, a knitted fabric produced by a tubular knitting method is preferred, which has a large packing weight per unit volume.

The single fiber diameter (hereinafter also referred to as fiber diameter) of the fiber (e.g., sea-island composite fiber) constituting the water-insoluble base material is not particularly limited, but from the viewpoints of improving the contact area with the substance to be adsorbed and maintaining the strength of the material, it is preferably from 3 to 200 μm, more preferably from 5 to 50 μm, and even more preferably from 10 to 40 μm. Any of the preferred lower limit values can be combined with any of the preferred upper limit values.

The term "single fiber diameter" means the average value obtained by taking 10 small samples of a fiber at random, photographing each at 1,000 to 3,000 magnifications using a scanning electron microscope, and measuring the diameter of the fiber at 10 points per each photograph (100 locations in total).

When the water-insoluble base material is in the form of a particles, the diameter of the particle is preferably 1 to 500 μm, from the viewpoint of ensuring a sufficient specific surface area for adsorbing the target substance.

Since blood purification material needs to interact with the substance to be adsorbed, it must have ligands bonded to at least its surface that comes into contact with organic matter contained in blood or the like.

The term "surface" refers to the surface of a water-insoluble base material, and in a case of a shape having pores on its surface, the surface also includes the outermost layer portion along the irregularities of the pores. Furthermore, in a case where the fiber has through-holes inside, the surface includes not only the outermost layer portion of the water-

insoluble base material, but also the outer layer of the through-holes inside the base material.

The term "bond" refers to a state in which a ligand is present on the surface of a water-insoluble base material through chemical interaction or physical interaction. Here, chemical interaction means a covalent bond, an electrostatic interaction, or a hydrogen bond, and physical interaction means van der Waals forces. Although not particularly limited, the bond between the water-insoluble base material and the ligand is preferably a covalent bond, since the covalent bond allows a ligand to stably exist on the surface when in contact with blood.

The term "ligand" refers to a compound bound to the surface of a water-insoluble base material, and its chemical structure is not particularly limited as long as it contains at least one acidic functional group or basic functional group. Examples of the ligand include ligands containing acidic functional groups and ligands containing basic functional groups. The functional group may be a combination of multiple identical or different functional groups. In addition, the ligand may further have a neutral functional group, so long as it has the above-mentioned acidic functional group or basic functional group. Examples of the neutral functional group include alkyl groups such as a methyl group and an ethyl group, and aryl groups such as a phenyl group, a phenyl group substituted with an alkyl group, and a phenyl group substituted with a halogen atom. Examples of the phenyl group substituted with an alkyl group include a para (p)-methylphenyl group, a meta (m)-methylphenyl group, an ortho (o)-methylphenyl group, a para (p)-ethylphenyl group, a meta (m)-ethylphenyl group, and an ortho (o)-ethylphenyl group. Examples of the phenyl group substituted with a halogen atom include a para (p)-fluorophenyl group, a meta (m)-fluorophenyl group, an ortho (o)-fluorophenyl group, a para (p)-chlorophenyl group, a meta (m)-chlorophenyl group, and an ortho (o)-chlorophenyl group. The neutral functional group and the acidic functional group or the basic functional group may be bonded directly or via a spacer (the spacer involved in the bond is referred to as spacer 1). Examples of the spacer 1 include a urea bond, an amide bond, and a urethane bond.

The water-insoluble base material and the ligand containing an acidic functional group or the ligand containing a basic functional group may be bonded directly or via a spacer derived from a reactive functional group (the spacer involved in the bond is referred to as spacer 2). The spacer 2 may be any one having an electrically neutral chemical bond such as a urea bond, an amide bond, an ether bond, an ester bond, or a urethane bond, and is preferably one having an amide bond or a urea bond. In the blood purification material of the present invention, spacer 2 is considered to be included in a ligand containing an acidic functional group or a ligand containing a basic functional group.

Reactive functional groups that mediate the bonds of the water-insoluble base material to a ligand containing acidic functional groups and to a ligand containing basic functional groups include an active halogen group such as a haloalkyl group (a halomethyl group, a haloethyl group, etc.), a haloacyl group (a haloacetyl group, a halopropionyl group, etc.) or a haloacetamidoalkyl group (a haloacetamidomethyl group, a haloacetamidoethyl group, etc.), an epoxide group, a carboxyl group, an isocyanate group, a thioisocyanate group, or an acid anhydride group. From the viewpoint of having an appropriate reactivity, the reactive functional group is preferably an active halogen group, more preferably a haloacetamidoalkyl group, and even more preferably a haloacetamidomethyl group. Specific examples of the water-insoluble base material having a reactive functional group introduced therein include polystyrene having a chloroacetamidomethyl group introduced on the surface thereof, and polysulfone having a chloroacetamidomethyl group introduced on the surface thereof.

The reactive functional group can be bonded to the water-insoluble base material by reacting the water-insoluble base material with an appropriate reagent in advance. For example, when the component constituting the water-insoluble base material is polystyrene and the reactive functional group is a chloroacetamidomethyl group, polystyrene having a chloroacetamidomethyl group bonded thereto can be obtained by reacting polystyrene with N-hydroxymethyl-2-chloroacetamide. Polystyrene having tetraethylenepentamine bonded thereto via an acetamidomethyl group is obtained by reacting polystyrene having a chloroacetamidomethyl group bonded thereto with, for example, tetraethylenepentamine having an amino group. In this case, the acetamidomethyl group corresponds to spacer 2, and tetraethylenepentamine corresponds to the ligand. The components constituting the water-insoluble base material, the spacers (spacer 1 and spacer 2), and the ligands can be combined arbitrarily. Examples of the water-insoluble base material having a ligand bonded thereto include polystyrene having a ligand containing a polyamine such as ethylenediamine, diethylenetriamine, triethylenetetramine, or tetraethylenepentamine bonded thereto via an acetamidomethyl group, and polysulfone having a ligand containing a polyamine such as ethylenediamine, diethylenetriamine, triethylenetetramine, or tetraethylenepentamine bonded thereto via an acetamidomethyl group.

The term "ligand containing an acidic functional group" means a compound that contains, as part of its chemical structure, at least one acidic functional group selected from the group consisting of a sulfuric acid group ($-OSO_2OH$), a sulfonic acid group ($-SO_2OH$), a carboxylic acid group (-COOH), and salts thereof. The chemical structure of the ligand is not restricted so long as it has the above-described acidic functional group. In this embodiment, however, when the component of the water-insoluble base material is polystyrene, sulfonic acid groups or salts thereof are preferred as acidic functional groups because they can be easily introduced into the benzene ring.

The term "ligand containing a basic functional group" means a compound that contains, as part of its chemical structure, at least one basic functional group selected from the group consisting of amino groups and salts thereof. There are no limitations on the chemical structure of the ligand as long as it has an amino group. However, polyamine is preferred

as a ligand containing a basic functional group, from the viewpoints that it can be easily introduced into water-insoluble base materials via an amide bond, a urea bond, or the like, and that one with a large MwB can be used.

The term "polyamine" refers to a compound having two or more amino groups as part of its chemical structure, and examples thereof include polyethyleneamines such as ethylenediamine, diethylenetriamine, triethylenetetramine, tetraethylenepentamine, pentaethylenehexamine, hexaethyleneheptamine, heptaethyleneoctamine, octaethylenenonamine, and polyethyleneimine. Tetraethylenepentamine is preferred as polyamine. The amino group in the polyamine structure is preferably an amino group derived from a primary amine or a secondary amine. The above-described polyamine may be linear, branched, or cyclic. Furthermore, the polyamine may contain an alkyl group having 1 to 10 carbon atoms, an unsaturated alkyl chain such as a vinyl group, or an allyl group; an aromatic substituent such as a phenyl group, a naphthyl group, or an anthracyl group; or a heterocyclic substituent such as an imidazolyl group, a pyridyl group, or a piperidyl group.

The mass average molecular weight (MwA) of a ligand containing an acidic functional group can be identified by preparing an extraction liquid by hydrochloric acid hydrolysis of the blood purification material and performing gel permeation chromatography (hereinafter, GPC). A specific example of the measurement method is as follows. The blood purification material cut into a 2 cm × 2 cm piece is placed in a vial, 2 mL of 6 M hydrochloric acid is added, and the material is then heated at 110°C for 20 hours using a dry heat sterilizer to prepare an extraction liquid. 0.5 mL of the resulting extraction liquid is taken, and 1 mL of water/methanol = 1/1 (with 0.1 N lithium nitrate added) is added, and further a cation exchange resin is added and allowed to stand. The supernatant after standing is used as a measurement solution. The obtained measurement solution is measured using a gel permeation chromatograph analyzer [for example, Prominence GPC system (manufactured by Shimadzu Corporation)] to identify MwA. An example of the configuration of the GPC system and the measurement conditions are as follows.

System configuration

Pump: LC-20AD
Auto sampler: SIL-20AHT
Column oven: CTO-20A
Detector: RID-10A
Column: GMPWXL (inner diameter, 7.8 mm × 30 cm; particle size, 13 μm), manufactured by Tosoh Corporation

Measurement condition

Measurement solvent: water/methanol = 1/1 (with 0.1 N lithium nitrate added)
Flow rate: 0.5 mL/min
Measurement time: 30 minutes
Amount of injected sample: 20 μL
Standard material for calibration curve: PEG/PEO standard sample (0.1 kDa to 1,258 kDa; manufactured by Agilent)

When the MwA is measured by GPC and no peak is observed on the obtained GPC chart, that is, when the MwA is determined to be less than 1,000, the MwA can be identified by performing gas chromatography-mass spectrometry (hereinafter, GCMS) instead of GPC. A specific measurement method is as follows. A blood purification material cut into a 2 cm × 2 cm piece is placed in a vial, 2 mL of 6 M hydrochloric acid is added, and the material is then heated at 110°C for 20 hours using a dry heat sterilizer to prepare an extraction liquid. 0.5 mL of the resulting extraction liquid is taken and 1.5 mL of water is added to prepare a measurement solution. The obtained measurement solution is measured using a gas chromatograph-mass spectrometer to identify MwA. An example of the configuration of the GCMS system and the measurement conditions are as follows.

System configuration

Gas chromatograph: GC-2010 (manufactured by Shimadzu Corporation)
Detector: GCMS-QP2010 Plus (manufactured by Shimadzu Corporation)
Column: DB-WAX (manufactured by Agilent)

Measurement conditions

Carrier gas: He 183 mL/min
Oven temperature: 40°C (Hold for 5 minutes) → 180°C (20 °C /min, Hold for 3 minutes)
Vaporizing chamber temperature: 200°C
Ion source temperature: 200°C
Interface temperature: 250°C
Injection method: Split ratio 20:1

The mass average molecular weight (MwB) of a ligand containing a basic functional group can be identified by hydrolyzing the blood purification material with hydrochloric acid to prepare an extraction liquid in the same manner as for the measurement of MwA, neutralizing the extraction liquid with a 6 M aqueous sodium hydroxide solution, and then performing GPC or GCMS in the same manner as for the measurement of MwA.

When MwA is too large, the motility is inhibited by forming an ion complex with a ligand containing a basic functional group, and the interaction with blood components is reduced. When MwA is too small and MwB is large, the ligands containing acidic functional groups will be covered with ligands containing basic functional groups, which inhibits interaction with blood components. For these reasons, MwA is preferably from $0.5 \times 10^2$ to $90.0 \times 10^3$, more preferably from $0.5 \times 10^2$ to $20.0 \times 10^3$, even more preferably from $0.5 \times 10^2$ to $1.3 \times 10^3$, still more preferably from $0.5 \times 10^2$ to $1.0 \times 10^3$, and most preferably from $0.7 \times 10^2$ to $0.9 \times 10^3$. The preferred lower limit value can be combined with any of the preferred upper limit values.

When MwB is too large, the motility is inhibited by forming an ion complex with a ligand containing an acidic functional group, and the interaction with blood components is reduced. When MwB is too small and MwA is large, the ligands containing basic functional groups will be covered with ligands containing acidic functional groups, which inhibits interaction with blood components. For these reasons, MwB is preferably from $1.0 \times 10^2$ to $66.0 \times 10^3$, more preferably from $1.0 \times 10^2$ to $22.0 \times 10^3$, even more preferably from $1.0 \times 10^2$ to $14.0 \times 10^3$, still more preferably from $1.0 \times 10^2$ to $13.0 \times 10^3$, and most preferably from $1.7 \times 10^2$ to $13.0 \times 10^3$. The preferred lower limit value can be combined with any of the preferred upper limit values.

The "value (MwA/MwB) obtained by dividing MwA by MwB" is calculated by dividing MwA by MwB. By setting MwA/MwB to less than 1.000, the adsorption ratio of multiple inflammatory cytokines having different surface charges can be improved. This is thought to be because the ligands containing acidic functional groups and the ligands containing basic functional groups can each develop interactions with blood components such as inflammatory cytokines without forming ion complexes. Furthermore, when MwA/MwB is 1.00 or more, it is believed that the ligands containing acidic functional groups form hydrogen bonds with each other, making it impossible for them to interact with blood components such as inflammatory cytokines. Therefore, MwA/MwB is preferably less than 1.000, more preferably 0.001 or more and less than 1.000, even more preferably 0.001 to 0.430, and most preferably 0.006 to 0.430. Any of the preferred lower limit values can be combined with any of the preferred upper limit values.

The content of the acidic functional group can be determined by acid-base back titration of the blood purification material. As a measurement method, for example, the surface of the blood purification material that has been previously measured for dry weight is desalted with hydrochloric acid, and then washed repeatedly with ion-exchanged water until the pH of the washing liquid becomes neutral. Subsequently, the material is dried by removing water by vacuum drying, and an aqueous sodium hydroxide solution of known concentration is added to convert the acidic functional groups on the surface of the material into salts. The concentration of sodium hydroxide consumed at this time can be measured by titration to determine the content of acidic functional groups.

When the content of acidic functional groups is too low, they will not be able to interact with blood components, and when the content is too high, they may develop adsorption performance to blood, which may increase blood coagulation. Therefore, the content of the acidic functional groups is preferably 0.02 to 3.00 mmol, more preferably 0.02 to 2.00 mmol, even more preferably 0.02 to 1.50 mmol, and most preferably 0.80 to 1.50 mmol per gram of the dry weight of the blood purification material. Any of the preferred lower limit values can be combined with any of the preferred upper limit values.

The content of the basic functional group can be determined by acid-base back titration of the blood purification material. As a measurement method, for example, the surface of the blood purification material that has been previously measured for dry weight is desalted with an aqueous sodium hydroxide solution, and then washed repeatedly with ion-exchanged water until the pH of the washing liquid becomes neutral. Subsequently, the material is dried by removing water by vacuum drying, and hydrochloric acid of known concentration is added to convert the basic functional groups on the surface of the material into salts. The concentration of hydrochloric acid consumed at this time can be measured by titration to determine the content of basic functional groups.

When the content of basic functional groups is too low, they will not be able to interact with blood components, and when the content is too high, they may exhibit hemolytic toxicity to blood, raising concerns about reduced safety. Therefore, the content of the basic functional groups is preferably 0.50 to 2.00 mmol, more preferably 0.50 to 1.50 mmol, even more preferably 0.70 to 1.50 mmol, and most preferably 1.10 to 1.50 mmol per gram of dry weight of the blood purification material. Any of the preferred lower limit values can be combined with any of the preferred upper limit values.

The blood purification material according to this embodiment is preferably used as a material to be packed into a blood purification column. Particularly, it is suitably used as a material for use in adsorbing and removing inflammatory cytokines when extracorporeal circulation is performed for the purpose of treating inflammatory diseases. When a blood purification column using the blood purification material is used for blood purification therapy, blood drawn from the body may be passed directly through the column, Alternatively, the column may be used in combination with a plasma separation membrane or the like.

The term "inflammatory disease" refers to all diseases that induce an inflammatory response in the body, and examples thereof include systemic lupus erythematosus, malignant rheumatoid arthritis, multiple sclerosis, ulcerative colitis, Crohn's disease, drug-induced hepatitis, alcoholic hepatitis, hepatitis A, hepatitis B, hepatitis C, hepatitis D, hepatitis E, sepsis (e.g., gram-negative sepsis, gram-positive sepsis, culture-negative sepsis, fungal sepsis), influenza, acute respiratory distress syndrome (ARDS, also known as acute tachypnea syndrome or acute respiratory promotion syndrome), acute lung injury (ALI), pancreatitis, idiopathic pulmonary fibrosis (IPF), inflammatory bowel disease (e.g., ulcerative colitis and Crohn's disease), reperfusion injury after organ transplantation, cholecystitis, cholangitis, and neonatal blood type incompatibility.

Among inflammatory diseases, the following are preferred targets for treatment with a blood purification column, since causative substances are released into the blood and blood purification is particularly expected to have a therapeutic effect: drug-induced hepatitis, alcoholic hepatitis, hepatitis A, hepatitis B, hepatitis C, hepatitis D, hepatitis E, sepsis (e.g., gram-negative bacterial sepsis, gram-positive bacterial sepsis, culture-negative sepsis, fungal sepsis), influenza, acute respiratory distress syndrome, acute lung injury, pancreatitis, and idiopathic pulmonary fibrosis. The blood purification column of this embodiment is preferably used, for example, in the treatment of the above-mentioned inflammatory diseases, and more preferably in the treatment of sepsis (e.g., gram-negative bacterial sepsis, gram-positive bacterial sepsis, culture-negative sepsis, fungal sepsis), influenza, acute respiratory distress syndrome, acute lung injury, and idiopathic pulmonary fibrosis, which are diseases that are thought to be difficult to treat with drugs alone and to involve inflammatory cytokines.

Examples of the method for evaluating the blood purification performance of the blood purification material include a method of measuring the IL-6 adsorption ratio and IL-8 adsorption ratio. IL-6 and IL-8 are types of inflammatory cytokines contained in blood components, and are known to significantly increase in the blood of patients with inflammatory diseases, making them suitable as blood components for evaluating blood purification performance. It can be determined that the higher the IL-6 adsorption ratio and the IL-8 adsorption ratio, the higher the blood purification performance of the blood purification material.

The cytokine adsorption performance of the blood purification material is preferably such that the IL-6 adsorption ratio is 10% or more and the IL-8 adsorption ratio is 20% or more, from the viewpoint of reducing the concentration of cytokines produced in the blood of patients with inflammatory diseases and enabling them to recover as quickly as possible from serious symptoms such as systemic shock and blood pressure reduction. Furthermore, it is more preferable that the IL-6 adsorption ratio is 10% or more and the IL-8 adsorption ratio is 40% or more, or that the IL-6 adsorption ratio is 20% or more and the IL-8 adsorption ratio is 20% or more, and it is even more preferable that the IL-6 adsorption ratio is 20% or more and the IL-8 adsorption ratio is 40% or more.

The blood purification column of the present invention is characterized by including the above-described blood purification material.

The term "blood purification column" refers to a column having at least a liquid inlet part, a case part, and a liquid outlet part, with the case part packed with a blood purification material. An example of the column is a radial flow type column.

The container shape of the blood purification column may be any shape that has an inlet part and an outlet part for a liquid containing blood components and the like (hereinafter referred to as liquid), as well as a case part, and that allows the case part to be packed with the blood purification material. One embodiment is a container that can be packed with blood purification material wrapped around a pipe and made into a cylindrical shape (hereinafter referred to as a cylinder), with liquid entering through the outer circumference of the cylinder, flowing to the inside of the cylinder, and then exiting the container, or with liquid entering the cylinder from the its inside, flowing to the outside of the cylinder, and then exiting the container. From the viewpoint of production efficiency and suppression of short-path flow of the treated liquid, the vessel of the blood purification column preferably has a structure in which the blood purification material is wrapped around a pipe having holes on the side surface. A specific example is a radial-flow type container including: a central pipe with holes provided on the side surface of the longitudinal direction for allowing the supplied liquid to flow out; a blood purification material that is packed around the central pipe and adsorbs target substances contained in the liquid; a plate connected to

the upstream end of the central pipe to allow the incoming liquid to pass through the central pipe and to prevent the liquid from coming into contact with the blood purification material without passing through the central pipe; and a plate arranged to seal the downstream end of the central pipe and fix the blood purification material to the space around the central pipe. The shape of the container may be a cylinder or a prismatic shape such as a triangular prism, a quadratic prism, a hexagonal prism or an octagonal prism, but is not limited to these shapes. Another possible embodiment is a container having a cylindrical space inside that can be packed with blood purification materials cut out in a circular shape and having a liquid feed port and a liquid discharge port. Specifically, examples include a container having a plate with a liquid feed port provided for feeding a liquid, and a plate with a liquid discharge port provided for discharging the fed liquid, and having a cylindrical case part packed with blood purification materials cut out in a circular shape. The shape of the blood purification material is not limited to a circular shape, but can be changed to any shape, such as an oval, a polygon such as a triangle or a square, or a trapezoid, depending on the shape of the container of the blood purification column.

Examples of the container for the blood purification column include containers made of glass, plastic/resin, and stainless steel, and containers made of plastic/resin are preferred in the viewpoint of handling in clinical sites or measurement locations and ease of disposal. The size of the container is selected appropriately depending on the intended use, but considering operation in clinical sites or measurement locations and ease of disposal, a size that is easy to hold in the hand is preferred, and it is preferable that the height of the entire blood purification column be 1 cm or more and 30 cm or less, the outer diameter be 1 cm or more and 10 cm or less, and the internal volume be 200 mL or less.

The blood purification material is preferably stacked and packed in the blood purification column. Here, the term "stacking" means stacking two or more sheets of blood purification material in close contact with each other. Examples of methods of stacking and packing include a method of stacking a plurality of sheets of blood purification material processed into sheet form, as in axial flow columns, and a method of wrapping a sheet of blood purification material around a pipe with holes, as in radial flow columns.

The blood purification column may be packed with the blood purification material alone, or in combination with other water-insoluble base materials and/or various spacers. Examples of spacers include knitted fabrics, woven fabrics, nonwoven fabrics, sheet-shaped fibers, membranes, beads, and hydrogels. Examples

The blood purification material of the present invention will be specifically described below with reference to examples, but the present invention is not limited to these examples.

(Example 1)

Polystyrene was used as a sea component and polypropylene was used as an island component. They were melt-metered separately and fed into a spin pack incorporating a sea-island composite spinneret having 700 distribution holes for the island component drilled per discharge hole to form a sea-island composite stream, which was then melt-discharged. The island ratio was controlled to 50 wt% and the distance from the surface of the sea-island composite fiber to the outermost island component was adjusted to 2 μm to obtain sea-island composite fibers with a single fiber fineness of 3.0 dtex (fiber diameter 20 μm).

The sea-island composite fibers were used to produce knitted fabric A having a basis weight of 60 g/m$^2$ and a bulk density of 0.20 g/mL by adjusting the stitch adjustment scale of a cylindrical knitting machine (model name: circular knitting machine MR-1, Maruzen Sangyo Co., Ltd.).

Eight grams of N-hydroxymethyl-2-chloroacetamide (hereinafter referred to as NMCA) was added to a mixed solution of 62 mL of nitrobenzene and 41 mL of 98 wt% sulfuric acid, and the mixture was stirred at 5°C until NMCA was dissolved, to prepare an NMCA solution. Next, 0.5 g of paraformaldehyde (hereinafter referred to as PFA) was added to a mixed solution of 5 mL of nitrobenzene and 3 mL of 98 wt% sulfuric acid, and the mixture was stirred at 50°C until the PFA was dissolved, to prepare a PFA solution. After 8 mL of the PFA solution was cooled to 5°C, it was mixed with 111 mL of the NMCA solution and stirred for 5 minutes. Five grams of knitted fabric A was immersed in a mixed solution of the PFA solution and the NMCA solution for 2 hours.

Next, knitted fabric A immersed in a mixed solution of the PFA solution and the NMCA solution was immersed in a mixed solution of 62 mL of nitrobenzene and 41 mL of 98 wt% sulfuric acid at 50°C for 4 hours. The knitted fabric A was immersed in 100 mL of nitrobenzene at 0°C to stop the reaction, and then separated by filtration using a glass filter and washed with 1,000 mL of methanol.

The knitted fabric A washed with methanol was immersed in a mixed solution of 1.3 mL of tetraethylenepentamine (TEPA; manufactured by Sigma-Aldrich; product number: 30-0850-05), 2 mL of triethylamine, and 93 mL of dimethyl sulfoxide (DMSO) at 40°C for 3 hours. The knitted fabric A was separated by filtration using a glass filter and washed with 1,000 mL of DMSO. The DMSO adhering to the knitted fabric was washed with 1,000 mL of methanol and 2,000mL of ion-exchanged water to obtain blood purification material 1.

(Example 2)

Based on Example 1 (method of producing blood purification material 1), blood purification material 2 was obtained in the same manner as in Example 1, except that the knitted fabric A was not immersed in a mixed solution of nitrobenzene and 98 wt% sulfuric acid and the amount of TEPA added was changed from 1.3 mL to 0.7 mL.

(Example 3)

Based on Example 1 (method of producing blood purification material 1), blood purification material 3 was obtained in the same manner as in Example 1, except that 1.3 mL of TEPA was replaced with 5 g of polyethyleneimine (manufactured by FUJIFILM Wako Pure Chemical Corporation; product number: 166-17825; average molecular weight: about 10,000 (catalog value)).

(Example 4)

Based on Example 1 (method of producing blood purification material 1), blood purification material 4 was obtained in the same manner as in Example 1, except that 1.3 mL of TEPA was replaced with 5 g of 30% polyethyleneimine P-70 solution (manufactured by FUJIFILM Wako Pure Chemical Corporation; product number: 169-11955; average molecular weight: about 70,000 (catalog value)).

(Comparative Example 1)

Knitted fabric A was produced in the same manner as in Example 1. Next, an NMCA solution was prepared in the same manner as in Example 1, and 5 g of the knitted fabric A was immersed in the solution for 2 hours. The knitted fabric A was immersed in 100 mL of nitrobenzene at 0°C to stop the reaction, and then separated by filtration using a glass filter and washed with 1,000 mL of methanol.

The knitted fabric A, which had been washed with methanol, was immersed in a mixed solution of 0.5 mL of ethylenediamine (manufactured by FUJIFILM Wako Pure Chemical Corporation) and 199 mL of DMSO at 40°C for 3 hours. The knitted fabric A was separated by filtration using a glass filter and washed with 200 mL of DMSO. The DMSO adhering to the knitted fabric A was washed with 1,000 mL of methanol.

The knitted fabric A was added directly to a 1.0 mass% polyacrylic acid 25000 solution, prepared by dissolving polyacrylic acid 25000 (manufactured by FUJIFILM Wako Pure Chemical Corporation; product number: 162-18581; average molecular weight: approximately 25000 (catalog value)) in 180 mL of methanol to 1.0 mass%, then 0.9 g of DMT-MM (manufactured by FUJIFILM Wako Pure Chemical Corporation) was further added thereto, and the fabric was immersed at 40°C for 2 hours. The knitted fabric A was separated by filtration using a glass filter and washed with 1,000 mL of methanol.

The knitted fabric A that had been washed with methanol was immersed in a mixed solution of 1.5 mL of TEPA and 93 mL of methanol at 40°C for 3 hours. The knitted fabric A was separated by filtration using a glass filter and washed with 1,000 mL of methanol and 2,000 mL of ion-exchanged water to obtain blood purification material 5.

(Comparative Example 2)

Based on Comparative Example 1 (method of producing blood purification material 1), blood purification material 6 was obtained in the same manner as in Comparative Example 1, except that 1.5 mL of TEPA was replaced with 5 g of polyethyleneimine (manufactured by FUJIFILM Wako Pure Chemical Corporation; product number: 166-17825; average molecular weight: about 10,000 (catalog value)).

(Example 5)

Based on Example 1 (method of producing blood purification material 1), blood purification material 7 was obtained in the same manner as in Example 1, except that the knitted fabric A was not immersed in a mixed solution of nitrobenzene and 98 wt% sulfuric acid and the amount of NMCA added was changed from 8 g to 6 g.

(Comparative Example 3)

Based on Comparative Example 1 (method of producing blood purification material 5), blood purification material 8 was obtained in the same manner as in Comparative Example 1, except that 1.5 mL of TEPA was replaced with 5 g of polyethyleneimine (branched) (manufactured by Sigma-Aldrich; product number: 408727; mass average molecular

weight: about 25,000 (catalog value)), and polyacrylic acid 25,000 was replaced with a polyacrylic acid solution (about 25%) 8,000 to 12,000 (manufactured by FUJIFILM Wako Pure Chemical Corporation; product number: 168-07375; molecular weight: about 150,000 (catalog value)).

(Comparative Example 4)

Based on Comparative Example 1 (method of producing blood purification material 5), blood purification material 9 was obtained in the same manner as in Comparative Example 1, except that the 1.0 mass% polyacrylic acid 25,000 solution was replaced with a sodium polyacrylate salt-containing solution (manufactured by FUJIFILM Wako Pure Chemical Corporation; product number: 192023; mass average molecular weight: about 2,000) to which 10 mL of 1 N hydrochloric acid had been added.

(Comparative Example 5)

Based on Comparative Example 1 (method of producing blood purification material 5), blood purification material 10 was obtained in the same manner as in Comparative Example 1 (method of producing blood purification material 5), except that 1.5 mL of TEPA was replaced with 5 g of polyethyleneimine (manufactured by FUJIFILM Wako Pure Chemical Corporation; product number: 167-17811; average molecular weight: about 1,800), 1.0 mass% polyacrylic acid 25,000 solution was replaced with a sodium polyacrylate salt-containing solution (manufactured by FUJIFILM Wako Pure Chemical Corporation; product number: 192023; mass average molecular weight: about 2,000) to which 10 mL of 1 N hydrochloric acid had been added.

<Measurement of MwA of blood purification materials by GPC>

The blood purification material cut into a 2 cm × 2 cm piece was placed in a vial, 2 mL of 6 M hydrochloric acid was added, and the material was then heated at 110°C for 20 hours using a dry heat sterilizer to prepare an extraction liquid. 0.5 mL of the resulting extraction liquid was taken, and 1.5 mL of water/methanol = 1/1 (with 0.1 N lithium nitrate added) was added, and further an anion exchange resin (manufactured by ORGANO CORPORATION, product number; IR120B H) was added and allowed to stand for 1 hour. The resulting supernatant after standing was used as the measurement solution. The resulting measurement solution was measured using a gel permeation chromatograph analyzer (Prominence GPC system; manufactured by Shimadzu Corporation) to identify MwA. The configuration of the GPC system and the measurement conditions are as follows.

System configuration

Pump: LC-20AD
Auto sampler: SIL-20AHT
Column oven: CTO-20A
Detector: RID-10A
Column: GMPWXL (inner diameter 7.8 mm × 30 cm, particle diameter 13 μm), manufactured by Tosoh Corporation

Measurement condition

Measurement solvent: water/methanol = 1/1 (with 0.1 N lithium nitrate added)
Flow rate: 0.5 mL/min
Measurement time: 30 minutes
Sample injection volume: 20 μL
Standard material for calibration curve: PEG/PEO standard sample (0.1 kDa to 1,258 kDa; manufactured by Agilent)

<Measurement of MwA of blood purification materials by GCMS>

When MwA of the blood purification material was measured by GPC and no peak was observed on the obtained GPC chart, MwA was measured by GCMS using the following method. The blood purification material cut into a 2 cm × 2 cm piece was placed in a vial, 2 mL of 6 M hydrochloric acid was added, and the material was then heated at 110°C for 20

hours using a dry heat sterilizer to produce an extraction liquid. 0.5 mL of the resulting extraction liquid was taken and 1.5 mL of water was added to make a measurement solution. The obtained measurement solution was measured using a gas chromatograph-mass spectrometer to identify MwA. An example of the configuration of the GCMS system and the measurement conditions are as follows.

System configuration

Gas chromatograph: GC-2010 (manufactured by Shimadzu Corporation)
Detector: GCMS-QP2010 (manufactured by Shimadzu Corporation)
Column: DB-WAX (manufactured by Agilent)

Measurement conditions

Carrier gas: He 183 mL/min
Oven temperature: 40°C (Hold for 5 minutes) → 180°C (20 °C /min, Hold for 3 minutes)
Vaporizing chamber temperature: 200°C
Ion source temperature: 200°C
Interface temperature: 250°C
Injection method: Split ratio 20:1

<Measurement of MwB of blood purification materials by GPC>

The blood purification material cut into a 2 cm × 2 cm piece was placed in a vial, 2 mL of 6 M hydrochloric acid was added, and the material was then heated at 110°C for 20 hours using a dry heat sterilizer to produce an extraction liquid. 0.5 mL of the resulting extraction liquid was taken and neutralized with 0.5 mL of a 6 M aqueous sodium hydroxide solution, and then 1 mL of water/methanol = 1/1 (with 0.1 N lithium nitrate added) was added, and further an anion exchange resin (manufactured by ORGANO CORPORATION, product number; HPR4780 Cl) was added and allowed to stand for 1 hour. The resulting supernatant after standing was used as the measurement solution. The obtained measurement solution was measured using a gel permeation chromatograph analyzer in the same manner as in measurement of MwA by GPC to identify MwB.

<Measurement of MwB of blood purification materials by GCMS>

When MwB of the blood purification material was measured by GPC and no peak was observed on the obtained GPC chart, MwB was measured by GCMS using the following method. The blood purification material cut into a 2 cm × 2 cm piece was placed in a vial, 2 mL of 6 M hydrochloric acid was added, and the material was then heated at 110°C for 20 hours using a dry heat sterilizer to produce an extraction liquid. 0.5 mL of the resulting extraction liquid was taken and neutralized with 0.5 mL of a 6M aqueous sodium hydroxide solution, and then 1 mL of water was added to prepare a measurement solution. The obtained measurement solution was measured using a gas chromatograph-mass spectrometer in the same manner as in measurement of MwA by GCMS to identify MwB.

<Measurement of acidic functional group content per gram of dry weight of blood purification material>

0.5 g of the blood purification material was placed in a 50 mL polypropylene centrifuge tube, 40 mL of 6 M hydrochloric acid was added, and the mixture was mixed by inversion at room temperature for 30 minutes. After mixing, only the solution was decanted, and 20 mL of ion-exchanged water was added, followed by mixing by inversion for 5 minutes. An operation of decanting only the solution after mixing, adding 20 mL of ion-exchanged water, and mixing by inversion for 5 minutes was repeated 10 times, and finally the pH of the decanted solution was confirmed to be 6 or more using litmus paper. The blood purification material after washing was placed in a vacuum dryer heated to 40°C and vacuum dried for 24 hours. The dried blood purification material was placed in a 50 mL polypropylene centrifuge tube, 40 mL of 0.1 M aqueous sodium hydroxide solution was added, and the mixture was mixed by inversion for 30 minutes. Five milliliters of the supernatant solution was removed from the centrifuge tube and placed in another 15 mL polypropylene centrifuge tube, and 5 mL of 0.1 M hydrochloric acid was further added, followed by the addition of 0.02 mL each of an aqueous methyl red solution and an aqueous phenolphthalein solution. Using a burette, 0.02 mL of 0.05 M aqueous sodium hydroxide

solution was dropped into the centrifuge tube, and the color tone of the solution was checked after mixing by inversion 10 times. Dropping of an aqueous sodium hydroxide solution, mixing the solution by inversion, and checking the color tone were repeated, and the amount of the aqueous sodium hydroxide solution dropped when the color of the solution was first observed to change from reddish orange to yellow was regarded as the titration amount. The content of acidic functional groups per gram of dry weight of the blood purification material was calculated using the following formula 1, and the obtained value was rounded off to two decimal places.

Acidic functional group content (mmol/g) per gram of dry weight of blood purification material = {volume of 0.1 M aqueous sodium hydroxide solution added (40 mL) / volume of aqueous sodium hydroxide solution removed (5 mL)} × titration amount per 1 g (mL/g) × concentration of aqueous sodium hydroxide solution (0.05 mol/L) Formula 1

<Measurement of basic functional group content per gram of dry weight of blood purification material>

0.5 g of the blood purification material was placed in a 50 mL polypropylene centrifuge tube, 20 mL of 6 M aqueous sodium hydroxide solution was added, and the mixture was mixed by inversion at room temperature for 30 minutes. After mixing, only the solution was decanted, and 20 mL of ion-exchanged water was added, followed by mixing by inversion for 5 minutes. An operation of decanting only the solution after mixing, adding 20 mL of ion-exchanged water, and mixing by inversion for 5 minutes was repeated 10 times, and finally the pH of the decanted solution was confirmed to be 8 or less using litmus paper. The blood purification material after washing was placed in a vacuum dryer heated to 40°C and vacuum dried for 24 hours. The dried blood purification material was placed in a 50 mL polypropylene centrifuge tube, 40 mL of 0.1 M hydrochloric acid was added, and the mixture was mixed by inversion for 30 minutes. Five milliliters of the supernatant solution was removed from the centrifuge tube and placed in another 15 mL polypropylene centrifuge tube, followed by the addition of 0.02 mL each of an aqueous methyl red solution and an aqueous phenolphthalein solution. Using a burette, 0.05M aqueous sodium hydroxide solution was dropped in 0.02 mL portions into the centrifuge tube, and the tube was mixed by inversion 10 times, after which the color of the solution was confirmed. Dropping of the aqueous sodium hydroxide solution, mixing by inversion, and checking the color tone were repeated, and the amount of the aqueous sodium hydroxide solution dropped when the color of the solution first changed from reddish orange to yellow was regarded as the titration amount per gram. The content (mmol/g) of basic functional groups per gram of dry weight of the blood purification material was calculated using the following formula 2, and the obtained value was rounded off to two decimal places.

Basic functional group content (mmol/g) per 1 g dry weight of blood purification material = {volume of 0.1 M hydrochloric acid added (40 mL) / volume of hydrochloric acid removed (5 mL)} × titration amount per 1 g (mL/g) × concentration of aqueous sodium hydroxide solution (0.05 mol/L) Formula 2

<Measurement of IL-6 adsorption ratio of blood purification material>

Six pieces of blood purification material, each cut out in the shape of a disc of 8 mm in diameter, were placed in a polypropylene assist tube. Fetal bovine serum (FBS) prepared to have an IL-6 concentration of 2,000 pg/mL was added to this assist tube in an amount converted to be 24 mL per 1 $cm^3$ of blood purification material (solid-liquid ratio: 0.04 $cm^3$/mL). After mixing by inversion in an incubator at 37°C for 1 hour, the IL-6 concentration in the FBS was measured by enzyme-linked immunosorbent assay (ELISA). The IL-6 adsorption ratio was calculated from the IL-6 concentrations before and after mixing by inversion according to the following formula 3. The value obtained by rounding from one decimal was used as an IL-6 adsorption ratio.

IL-6 adsorption ratio (%) = {IL-6 concentration (pg/mL) before mixing by inversion - IL-6 concentration (pg/mL) after mixing by inversion} / IL-6 concentration (pg/mL) before mixing by inversion × 100 Formula 3

<Measurement of IL-8 adsorption ratio of blood purification materials>

Four pieces of blood purification material, each cut out in the shape of a disc of 6 mm in diameter, were placed in a polypropylene assist tube. FBS prepared to have an IL-8 concentration of 2,000 pg/mL was added to this assist tube in an amount converted to be 286 mL per 1 $cm^3$ of blood purification material (solid-liquid ratio: 0.004 $cm^3$/mL). After mixing by inversion in an incubator at 37°C for 1 hour, the IL-8 concentration in the FBS was measured by ELISA. The IL-8 adsorption ratio was calculated from the IL-8 concentrations before and after mixing by inversion according to the following formula 4. The value obtained by rounding from one decimal was used as an IL-8 adsorption ratio.

IL-8 adsorption ratio (%) = {IL-8 concentration (pg/mL) before mixing by inversion - IL-8 concentration (pg/mL) after mixing by inversion} / IL-8 concentration (pg/mL) before mixing by inversion $\times$ 100     Formula 4

The MwA, MwB, content of acidic functional groups, content of basic functional groups, IL-6 adsorption ratio, and IL-8 adsorption ratio of blood purification materials 1 to 9 were measured, and the results are shown in Table 1.

[Table 1]

[Table 1]

| Example | Blood purification material | Molecular weight of ligand containing acidic functional group: MwA | Content of acidic functional group [mmol/g] | Molecular weight of ligand containing basic functional group: MwB | Content of basic functional group [mmol/g] | MwA/MwB | IL-6 Adsorption ratio [%] | IL-8 Adsorption ratio [%] |
|---|---|---|---|---|---|---|---|---|
| Example 1 | Blood purification material 1 | $0.8 \times 10^2$ | 1.44 | $1.9 \times 10^2$ | 1.50 | 0.421 | 84 | 93 |
| Example 2 | Blood purification material 2 | $0.8 \times 10^2$ | 0.05 | $1.9 \times 10^2$ | 0.70 | 0.421 | 80 | 30 |
| Example 3 | Blood purification material3 | $0.8 \times 10^2$ | 1.15 | $12.5 \times 10^3$ | 1.50 | 0.006 | 28 | 75 |
| Example 4 | Blood purification material 4 | $0.8 \times 10^2$ | 1.23 | $65.8 \times 10^3$ | 1.40 | 0.001 | 10 | 55 |
| Comparative Example 1 | Blood purification material 5 | $12.2 \times 10^3$ | 0.64 | $1.9 \times 10^2$ | 0.90 | 64.211 | 14 | 22 |
| Comparative Example 2 | Blood purification material 6 | $19.2 \times 10^3$ | 0.79 | $13.3 \times 10^3$ | 1.00 | 1.444 | 6 | 0 |
| Example 5 | Blood purification material 7 | $0.8 \times 10^2$ | 0.02 | $1.9 \times 10^2$ | 1.15 | 0.421 | 64 | 43 |
| Comparative Example 3 | Blood purification material 8 | $89.8 \times 10^3$ | 0.32 | $21.4 \times 10^3$ | 0.63 | 4.196 | 1 | 2 |
| Comparative Example 4 | Blood purification material 9 | $1.0 \times 10^3$ | 0.51 | $1.9 \times 10^2$ | 0.90 | 5.263 | 4 | 7 |

The results in Table 1 demonstrate that blood purification materials 1 to 4 and 7, each having MwA/MwB of less than 1.000, i.e., blood purification materials in which MwA is smaller than MwB, have high adsorption ratios for multiple cytokines (IL-6 and IL-8) with different surface charges. In contrast, blood purification materials 5, 6, 8, and 9, in which MwA is greater than MwB, are found to have low adsorption ratios for multiple cytokines with different surface charges.

Industrial Applicability

The blood purification material of the present invention can adsorb inflammatory cytokines at high efficiency, and can therefore be used as an adsorption material for use in extracorporeal circulation.

## Claims

1. A blood purification material, having a water-insoluble base material, a ligand containing an acidic functional group, and a ligand containing a basic functional group,

   wherein the ligand containing the acidic functional group and the ligand containing the basic functional group are bonded to the water-insoluble base material, and
   the value (MwA/MwB) obtained by dividing the molecular weight (MwA) of the ligand containing the acidic functional group by the molecular weight (MwB) of the ligand containing the basic functional group is less than 1.000.

2. The blood purification material according to claim 1, wherein the MwA/MwB is 0.001 to 0.430.

3. The blood purification material according to claim 1 or 2, wherein the MwA is $0.5 \times 10^2$ to $90.0 \times 10^3$, and the MwB is $1.0 \times 10^2$ to $66.0 \times 10^3$.

4. The blood purification material according to any one of claims 1 to 3, wherein the content of the acidic functional group is 0.02 to 3.00 mmol per gram of dry weight, and the content of the basic functional group is 0.50 to 2.00 mmol per gram of dry weight.

5. The blood purification material according to any one of claims 1 to 3, wherein the content of the acidic functional group is 0.02 to 1.50 mmol per gram of dry weight, and the content of the basic functional group is 0.70 to 1.50 mmol per gram of dry weight.

6. The blood purification material according to any one of claims 1 to 5, for use in adsorbing inflammatory cytokines.

7. A blood purification column comprising the blood purification material according to any one of claims 1 to 6.

**INTERNATIONAL SEARCH REPORT**

International application No. **PCT/JP2024/037771**

**A. CLASSIFICATION OF SUBJECT MATTER**

***A61M 1/36***(2006.01)i; ***B01J 20/26***(2006.01)i
FI: A61M1/36 165; B01J20/26 H

According to International Patent Classification (IPC) or to both national classification and IPC

**B. FIELDS SEARCHED**

Minimum documentation searched (classification system followed by classification symbols)

A61M1/36; B01J20/26

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2012/057185 A1 (TORAY INDUSTRIES, INC.) 03 May 2012 (2012-05-03) | 1-7 |
| A | JP 2023-81326 A (TORAY INDUSTRIES, INC.) 09 June 2023 (2023-06-09) | 1-7 |
| A | JP 2020-92879 A (TORAY INDUSTRIES, INC.) 18 June 2020 (2020-06-18) | 1-7 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2024** | **03 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/037771**

| Patent document cited in search report | Publication date (day/month/year) | Patent family member(s) | Publication date (day/month/year) |
|---|---|---|---|
| WO 2012/057185 A1 | 03 May 2012 | US 2013/0220912 A1 | |
| | | EP 2633872 A1 | |
| | | CA 2814942 A1 | |
| | | CN 103167886 A | |
| | | KR 10-2013-0056313 A | |
| JP 2023-81326 A | 09 June 2023 | (Family: none) | |
| JP 2020-92879 A | 18 June 2020 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5374064 B
- JP 2023121738 A
- JP 4579916 B